# EUROPÄISCHE PATENTSCHRIFT

(11) **EP 1 450 827 B1**
(45) Veröffentlichungstag und Bekanntmachung des Hinweises auf die Patenterteilung: **07.04.2010**
(21) Anmeldenummer: 02735207.9
(22) Anmeldetag: 02.04.2002
(51) Int. Cl.: A61L 24/10, C12N 5/071, C12N 5/10

(54) **VERWENDUNG VON ZWEI-KOMPONENTEN ZUSAMMENSETZUNGEN ZUR IN SITU HERSTELLUNG VON FIBROBLASTEN UND KERATINOZYTEN UMFASSENDEN ZELLTRANSPLANTATEN**
USE OF TWO-CONSTITUENT COMPOSITIONS FOR THE IN SITU PRODUCTION OF CELL TRANSPLANTS THAT COMPRISE FIBROBLASTS AND KERATINOCYTES
UTILISATION DES COMPOSITIONS A DEUX CONSTITUANTS POUR LA PRODUCTION IN SITU DE GREFFONS CELLULAIRES COMPRENANT DES FIBROBLASTES ET DES KERATINOCYTES

(30) Priorität: 02.04.2001 DE 10116362
(43) Veröffentlichungstag der Anmeldung: 01.09.2004
(73) Patentinhaber: Hartmann, Anke, Dr., 97080 Würzburg (DE); Friedl, Peter, Prof. Dr., 97076 Würzburg (DE)
(72) Erfinder: HARTMANN, Anke, 97080 Würzburg (DE); FRIEDL, Peter, 97076 Würzburg (DE)
(74) Vertreter: Gosdin, Michael
(86) Internationale Anmeldenummer: PCT/EP2002/003640
(87) Internationale Veröffentlichungsnummer: WO 2002/078721

(56) Entgegenhaltungen:
- EP-A- 1 184 040
- WO-A-00/25583
- WO-A-00/32207
- US-A1- 2001 006 813
- LAM P K ET AL: "Dermal fibroblasts do not enhance the graft take rate of autologous, cultured keratinocyte suspension on full-thickness wounds in rats." ANNALS OF PLASTIC SURGERY. UNITED STATES FEB 2001, Bd. 46, Nr. 2, Februar 2001 (2001-02), Seiten 146-149, XP001085443 ISSN: 0148-7043
- ROSENTHAL F M ET AL: "Paracrine stimulation of keratinocytes in vitro and continuous delivery of epidermal growth factor to wounds in vivo by genetically modified fibroblasts transfected with a novel chimeric construct." IN VIVO (ATHENS, GREECE) GREECE 1997 MAY-JUN, Bd. 11, Nr. 3, Mai 1997 (1997-05), Seiten 201-208, XP002075251 ISSN: 0258-851X
- HORCH RAYMUND E ET AL: "Single-cell suspensions of cultured human keratinocytes in fibrin-glue reconstitute the epidermis." CELL TRANSPLANTATION, Bd. 7, Nr. 3, Mai 1998 (1998-05), Seiten 309-317, XP002204016 ISSN: 0963-6897
- STARK G B ET AL: "[Biological wound tissue glue systems in wound healing]" LANGENBECKS ARCHIV FUR CHIRURGIE. SUPPLEMENT. KONGRESSBAND. DEUTSCHE GESELLSCHAFT FUR CHIRURGIE. KONGRESS. GERMANY 1998, Bd. 115, 1998, Seiten 683-688, XP001085344 ISSN: 0942-2854
- MEANA A ET AL: "Large surface of cultured human epithelium obtained on a dermal matrix based on live fibroblast-containing fibrin gels." BURNS, Bd. 24, Nr. 7, November 1998 (1998-11), Seiten 621-630, XP002204017 ISSN: 0305-4179
- PELLEGRINI G ET AL: "CULTIVATION OF HUMAN KERATINOCYTE STEM CELLS: CURRENT AND FUTURE CLINICAL APPLICATIONS" MEDICAL AND BIOLOGICAL ENGINEERING AND COMPUTING, PETER PEREGRINUS LTD. STEVENAGE, GB, Bd. 36, Nr. 6, 1. November 1998 (1998-11-01), Seiten 778-790, XP000784853 ISSN: 0140-0118
- CHAN ERIC S Y ET AL: "A new technique to resurface wounds with composite biocompatible epidermal graft and artificial skin." JOURNAL OF TRAUMA INJURY INFECTION AND CRITICAL CARE, Bd. 50, Nr. 2, Februar 2001 (2001-02), Seiten 358-362, XP001085434 ISSN: 1079-6061
- HANSBROUGH J F ET AL: "BURN WOUND CLOSURE WITH CULTURED AUTOLOGOUS KERATINOCYTES AND FIBROBLASTS ATTACHED TO A COLLAGEN GLYCOSAMINOGLYCAN SUBSTRATE" JAMA (JOURNAL OF THE AMERICAN MEDICAL ASSOCIATION), Bd. 262, Nr. 15, 1989, Seiten 2125-2130, XP000971590 ISSN: 0098-7484

## Beschreibung

Die vorliegende Erfindung betrifft die Gebiete der eukaryotischen Zellkultur und des Tissue-Engineering. Die Erfindung betrifft die Verwendung fibrinhaltiger Keratinozyten- und Fibroblastensuspensionen auf Zwei-Komponentenbasis in der Behandlung von Patienten mit chronischen Ulzera unterschiedlicher Genese (venös, arteriell, diabetisch, neuropathisch), Verbrennungswunden, posttraumatischen Wunddefekten nach Dermabrasion, Exzision oder Laserablation von Hautdetekten, sowie in der Defektdeckung ausgedehnter gutartiger nävoider Hautveränderungen (epidermale Nävi, Nävus papillomatosus et pilosus) und Narben (hypertrophe Narben und Keloide) nach vorheriger Dermabrasion.

Der medizinisch-biotechnologische Forschungs- und Anwendungsbereich des *Tissue Engineering* stellt eine in den letzten Jahren immer intensiver genutzte Möglichkeit dar, verletzte bzw. fehlende Gewebestrukturen bzw. Organe durch biotechnologisch hergestellte Transplantate zu ersetzen. In diesem Zusammenhang liegt ein Schwerpunkt auf der Herstellung und Transplantation sogenannter autologer Transplantate. Das Prinzip dieser Transplantationstechnik besteht darin, dass einem Patienten Zellen des gewünschten Typs (z.B. Keratinozyten) aus einem intakten Gewebebereich (Spenderareal) entnommen, anschließend *in vitro* kultiviert ("expandiert") und eventuell auf einen Träger aufgebracht und schließlich in das Empfängerareal des Patienten transplantiert werden.

Prinzipiell sind jedoch autologe Transplantate aus einer Vielzahl von Zelltypen herstellbar, beispielsweise aus Haut-, Knochen-, Knorpel- und Bindegewebe, sowie weiteren Gewebetypen.

Weiterhin existieren dermatologische Indikationsgebiete, die häufig die Transplantation von großflächigen Hautarealen erfordern. Hierbei sind als wichtigste Indikationen insbesondere Brandverletzungen, posttraumatische Wunddefekte, nävoide Hautveränderungen, Narben und Keloide, sowie die aufgrund der erhöhten Lebenserwartung der Bevölkerung zunehmend an Bedeutung gewinnenden chronischen Ulzera unterschiedlicher Genese zu nennen.

Die bisherigen Therapiemaßnahmen bei der Transplantation von Haut basieren im wesentlichen auf den im folgenden vorgestellten Transplantationsmaterialien bzw. Verfahren.

Zur Therapie von Verbrennungen wurden von O'Connor und Mulliken (Lancet, 1981, Vol. 10, Seiten 75-78) kultivierte Keratinozyten als mehrschichtige Epidermis (sog. Cultured epidermal autografts = CEA) erstmals 1981 erfolgreich angewandt. Als epidermaler mehrschichtiger Hautersatz für Patienten mit lebensbedrohlichen Verbrennungen ist seit 1987 Epicel™ der Fa. Genzyme in USA, Kanada, Deutschland, Frankreich und Japan arzneimittelrechtlich zugelassen. Die mögliche Verwendung einer präformierten mehrschichtigen Epidermis wird jedoch durch eine lange Kulturdauer von ca. 4-5 Wochen vor Transplantation eingeschränkt, wobei zugleich die zu erzielende mögliche maximale Deckungsfläche (2 cm² Biopsie entsprechen ca. 30 cm² Transplantatfläche) begrenzt ist. Die dünnen und mechanisch empfindlichen Transplantate stellen hohe Anforderungen an den Operateur und erfordern einen gut vorbereiteten Wundgrund. Außerdem sind die Zellen bereits differenziert und daher nur noch eingeschränkt proliferationsfähig. Dies führt häufig zu mechanisch instabilen Regeneraten und der Notwendigkeit wiederholter Retransplantationen (Cooper, 1993). Die in diesem Verfahren auch verwendeten autologen Keratinozyten, die aus 50- 100 Haarwurzeln (E-pigraft, Fa. BioCare GmbH, Leipzig) gewonnen werden können, sind erst nach 6 Wochen in Form von Zellpellets verfügbar, welche jedoch keine vollständige Wundabdeckung erreichen.

Weitere aus dem Stand der Technik bekannte Transplantate stellen sogenannte Keratinozyten-Fibrinmatrix-Konstrukte dar. Die Verwendung subkonfluent kultivierter Keratinozyten erfolgt durch die Kombination bzw. Mischung proliferativ aktiver Zellsuspension mit einer Fibrinsuspension. Das Zell-Fibringemisch wird im Wundbett vorübergehend fixiert, so dass die Keratinozyten im Wundbett migrieren, proliferieren, sich im dreidimensionalen Fibrinnetz ausrichten und unter Umbau der Fibrinmatrix eine mehrschichtige Epidermis *in situ* rekonstituieren können. Die erste klinische Anwendung autologer Keratinozyten als Zellsuspension in Fibrin erfolgte 1988 durch Hunyadi et al. (J. Dermatol. Surg. Oncol., 1988, Vol. 14, Seiten 75-78). Bei 16 von 20 Patienten mit Ulcera bis 25 cm² konnte eine vollständige Reepithelisierung nach nur 2 Wochen erreicht werden. Seit 1993 wird die Keratinozyten-Fibrin-Glue-Suspension (KFGS) in der Chirurgischen Universitätsklink Freiburg, Abteilung Plastische und Handchirurgie, vornehmlich bei großflächigen Brandverletzungen (s. auch Stark et al., Eur. J. Plast. Surg., 1995, Vol. 18, Seiten 267-271) angewandt. Im Mausmodell zeigten KFGS-Transplantate im Vergleich zu präformierten mehrschichtigen Transplantaten (CEA, s.o.) postoperativ eine höhere mittlere Epidermisdicke und eine intakte Basalmembran mit Expression von Kollagen Typ VII, welches in der CEA-Gruppe fehlte (Horch et al., Cell Transplant., 1998, Vol. 7, Seiten 309-317). Außerdem waren bei Verbrennungspatienten, wenn CEA zur Anwendung kam, im Vergleich zur KFGS-Transplantation häufigere Retransplantationen notwendig (Stark et al., Eur. J. Plast. Surg., 1995, Vol. 18, Seiten 267-271).

Desweiteren wurden zur Transplantation dermo-epidermale Allotransplantate verwendet, d.h. Transplantate, bei denen allogene Spenderzellen in einer alloplastischen oder xenogenen Stützmatrix verwendet werden. In der EU ist seit 1994 als dermales Allotransplantat bislang AlloDerm® der Fa. LifeCell Corporation zugelassen, welches aus de-epidermisierter und dezellularisierter Spenderhaut Verstorbener besteht (Wainwright et al., J. Burn Care Rehabil., 1996, Vol. 17, Seiten 124-136). Weiterhin ist das Produkt TransCyte® erhältlich, welches aus Vorhautfibroblasten in einem nicht resorbierbaren 3D-Nylongitter besteht und der temporären Defektdeckung dient, sowie Dermagraft®, bei welchem statt Nylon ein resorbierbares Vicrylgitter verwendet wird. Als erstes und bislang einziges zweischichtiges Komposithaut-Transplantat ist Apligraf® (Graftskin) der Fa. Organogenesis/ Novartis Pharma AG seit Mai 1998 in USA, Canada und in der Schweiz zugelassen. Hierbei werden in vitro Vorhautfibroblasten in Suspension mit bovinem Typ I Kollagen polymerisiert. Anschliessend werden auf die kontrahierte Kollagenmatrix allogene Keratinozyten (aus Vorhäuten Neugeborener) ausgesät, die schliesslich durch entsprechende Kulturbedingungen bereits *in vitro* eine vollständig differenzierte und verhornende Epidermisoberfläche bilden (Muhart et al., Lancet, 1997, Vol. 350, Seite 1142).

Allerdings ergeben sich trotz der mit den oben dargestellten Verfahren erzielten Erfolge eine Reihe von bislang ungelösten Schwierigkeiten bzw. Nachteilen. Präformierte mehrschichtige epidermale Transplantate sind, da sie bereits differenziert sind, nur noch eingeschränkt proliferationsfähig und mechanisch instabil (s. z.B. CEA).

Allotransplantate (aus homologen Spenderzellen) weisen zum einen das Problem eines gewissen Infektionsrisikos auf. Weiterhin wird üblicherweise bei der Kultivierung der zu transplantierender Zellen fetales Kälberserum (FCS) sowie als Matrix bovines Kollagen verwendet, wobei ein gewisses Infektionsrisiko mit BSE nach dem derzeitigen Stand der Wissenschaft nicht völlig ausgeschlossen werden kann. Die im Stand der Technik üblichen Dermisäquivalente weisen weiterhin eine präformierte Kollagenstruktur auf, die sich dem Wundareal häufig nicht optimal mechanisch und physiologisch anpassen kann, so daß kosmetisch unbefriedigende Transplantationsergebnisse erhalten werden oder sogar eine Abstoßung des Transplantates stattfindet. Außerdem ergibt sich aufgrund der Dicke der extrazellulären Matrix, insbesondere bei alloplastischen Materialien (z.B. bei 3 D-Silikon-, -Nylon-, -Vicrylgittern), aber auch bei Kollagen-Gittern, eine Beeinträchtigung der Diffusionsstrecke von Nährlösungen bzw. Nährstoffen und damit der initialen Versorgung der Keratinozyten nach der Transplantation mit dem Risiko von Nekrosen. Dies ist vor allem auf eine mangelhafte bzw. gestörte dermo-epidermale Interaktion zurückzuführen, die die Neubildung bzw. Proliferation verschiedener Zelltypen (Fibroblasten und Keratinozyten) erfordert. Letztendlich führen diese Probleme zu einer verminderten mechanischen Stabilität des präformierten Transplantates und damit zu einem höheren Risiko einer mißlungenen Transplantation.

WO 00/32207 A1 offenbart ein Verfahren, bei dem nach einer Beschichtung einer Wundoberfläche (coating) mit einem Sprühverfahren ein Gemisch, bestehend aus Fibroblasten, Keratinozyten sowie einem Fibrinvorläufer, auf die Wunde aufgetragen wird. Die zeitlich getrennte und bewusst aufeinander abgestimmter Auftragung der Zellarten unter Berücksichtigung spezifischer Zelleigenschaften, die im Verlauf der Wundheilung relevant sind, ist in WO 00/32207 A1 nicht vorgesehen.

Dokument EP 1 184 040 A1 offenbart Hautersatzmaterialien (Transplantate), die neben humaner Haut, die als ein weitlumig expandiertes Netz aufgearbeitet ist, zusätzlich verschiedene hautspezifische Zellen (Keratinozyten, Melanozyten, Fibroblasten und/oder Endothelzellen) aufweisen. Das Dokument EP 1 184 040 A1 ist als Stand der Technik nach Art. 54 (3) EPÜ einzustufen.

Meana et al. (Large surface of cultured human epithelium obtained on a dermal matrix based on live fibroblast-containing fibrin gels, BURNS,Vol. 24, No. 7, November 1998, 621-630) offenbart ein in-vitro präformiertes Composite-Graft. Bei dem Composite-Graft handelt es sich somit um einen Hautersatz in Form eines Transplantates, der in einem Nährmedium außerhalb der Wunde, also außerhalb des menschlichen Körpers vorgefertigt wird.

Die gleichzeitige Applikation von Fibroblasten und Keratinozyten, neben dem Einsatz weiterer hautspezifischer Zellen und Komponenten, offenbaren auch verschiedene wissenschaftliche Publikationen (Lam et al., Dermal fibroblasts do not enhance the graft take rate of autologous, cultured keratinocyte suspension on full-thickness suspension on full-thickness wounds in rats, Annals of plastic surgery, 2001, Vol. 46, No 2, 146-149; Rosenthal et al., Paracrine stimulation of keratinocytes in vitro and continuous delivery of epidermal growth and continuous delivery of epidermal growth factor to wounds in vivo by genetically modified fibroblasts transfected with a cimeric construct", In vivo, 1997, Vol. 11, No. 3, 201-208; Horch et al,, Single-cell suspensions of cultured human keratinocytes in fibrin-glue reconstitute the epidermis, Cell transplantation, 1998, Vol. 7, No. 3, 309-317; Stark et al., Biological wound tissue glue systems in wound healing, Langenbecks Archiv für Chirurgie Supp. II, Kongressbericht 1998, Deutsche Gesellschaft für Chirurgie, 1998, Vol. 115, 638-688). Die genannten Publikationen, die u.a. auch auf außerhalb der Wunde gefertigte Transplantate gerichtet sind, offenbaren jedoch nicht die zeitlich getrennte und bewusst aufeinander abgestimmte Auftragung von hautspezifischen Zellarten unter Berücksichtigung spezifischer Zelleigenschaften, die im Verlauf der Wundheilung relevant sind.

Demzufolge ist es bisher nicht gelungen, ein Hauttransplantat zu entwickeln, das mechanische Festigkeit mit einer optimalen physiologischen und mechanischen Anpassung an das Wundareal bei gleichzeitig geringem Infektionsrisiko verbindet und das in akuten Fällen innerhalb kurzer Zeiträume zur Verfügung steht. Ferner existiert im Stand der Technik bis jetzt kein Transplantat, das auch in der Lage ist, bei profunden Verletzungen auch tiefere Wunden mit Granulationsgewebe auszufüllen, was dazu führt, daß bei tieferen Wunden zunächst die "natürliche" Ausfällung der Wunde mit körpereigenem Granulationsgewebe abgewartet werden muß, bevor z.B. eine Keratinozytensuspension zur Abdeckung des Oberflächenareals aufgebracht werden kann. Weiterhin enthalten die bisher verwendeten Transplantate des Standes der Technik Kollagen, was dazu führt, daß die Synthese von Kollagen und physiologischem Granulationsgewebe in der Wunde gehemmt bzw. erschwert wird.

Folglich bestand eine Aufgabe der vorliegenden Erfindung in der Schaffung eines Transplantatsystems, das ein geringes Infektionsrisiko für den Patienten darstellt. Eine weitere Aufgabe der vorliegenden Erfindung bestand darin, ein Transplantatsystem bereitzustellen, das eine optimale physiologische und mechanische Anpassung des Transplantates an das Wundareal erlaubt, wobei neben oberflächlichen Wunden insbesondere erstmals die Behandlung von profunden Wunden unter Verwendung von fibrin- bzw. fibrinogenhaltigen Suspensionen autologer Zellen (Fibroblasten und Keratinozyten) ermöglicht werden sollte. Schließlich bestand eine weitere Aufgabe der vorliegenden Erfindung in der Bereitstellung von kollagenfreien Zellsuspensionen (Keratinozyten und Fibroblasten) zur Verwendung als autologe Transplantate.

Demgemäß betrifft die vorliegende Erfindung eine Zwei-Komponenten Zusammensetzung zur Verwendung in der Behandlung von Hautdefekten, Ulzera, und Wunden umfassend die folgenden Bestandteile:
a) eine Fibroblastensuspension (Komponente a) enthaltend mindestens die getrennt vorliegenden Fibrinkleberbestandteile Thrombin und Calciumchlorid (Fibrinkleberbestandteil 1) und Fibrinogen (Fibrinkleberbestandteil 2) und
b) eine Keratinozytensuspension (Komponente b) enthaltend mindestens die getrennt vorliegenden Fibrinkleberbestandteile Thrombin und Calciumchlorid (Fibrinkleberbestandteil 1) und Fibrinogen (Fibrinkleberbestandteil 2).

Die Zwei-Komponenten Zusammensetzung gemäß der vorliegenden Erfindung ist insbesondere dadurch charakterisiert, dass kein präformiertes Transplantat verwendet wird, wie z.B. bei der CEA-Transplantation, sondern dass ein System bestehend aus den zwei Suspensionen (Komponenten) a) und b) zur Wundheilung eingesetzt wird. Dies hat gegenüber den vorgeformten Transplantaten auf der Basis von konfluent kultivierten Zellen entscheidende Vorteile. So bewirkt die erfindungsgemäß verwendete Zusammensetzung aus Fibroblasten und Keratinozyten, dass *in situ* eine verbesserte dermo-epidermale Interaktion auftritt. Für die mechanische Stabilität einer dermo-epidermalen Junktionszone ist die Ausbildung einer kontinuierlichen, nicht fragmentierten Lamina densa sowie die Bildung von Verankerungsfilamenten ("anchoring fibrils") von besonderer Bedeutung. Durch die mit der erfindungsgemäßen Zwei-Komponenten Zusammensetzung erreichte Koinkubation von Keratinozyten mit Fibroblasten *in situ* wird die Entwicklung nativer extrazellulärer Matrixstrukturen ermöglicht. Das Prinzip der Synthese einer dermo-epidermalen Junktionszone im Sinne eines physiologischen Basalmembran-Äquivalents durch die Zellen selbst wird daher durch die Verwendung der erfindungsgemäßen Zusammensetzung realisiert. Desweiteren bietet die Verwendung der erfindungsgemäßen Zwei-Komponenten Zusammensetzung den Vorteil, daß die bei präformierten Transplantaten des Standes der Technik auftretenden mechanischen Probleme bei der Transplantation, wie z.B. Zerreißen oder Verkleben des Transplantates, nicht auftreten können. Die für die Zellen gemäß (a) und (b) verwendeten Suspensionskulturen werden vorzugsweise unter Reinraumbedingungen nach GMP-Standards hergestellt. Für den gesamten Herstellungsprozeß der erfindungsgemäßen Zusammensetzung sind Bedingungen der Reinheitsklasse A gemäß GMP-Standard bevorzugt, d.h., es dürfen nicht mehr als 3500 Partikel, die mindestens 0,5 µm groß sind und 0 Partikel, die mindestens 5 µm groß sind, pro Kubikmeter Luft vorhanden sein (bezügl. der Anforderungen an bestimmte Reinheitsklassen siehe "EG-Leitfaden einer guten Herstellungspraxis für Arzneimittelmit Betriebsverordnung für pharmazeutische Unternehmer" (1998), 5. Auflage, Hrsg.: G. Auterhoff; Editio Cantor Verlag, Aulendorf). Die weiteren Verfahrensschritte zur Herstellung von Suspensionskulturen sind dem Fachmann bekannt. Die Bestandteile des Zwei-Komponenten Systems bestehend aus (a) und (b) umfassen neben den suspendierten Fibroblasten bzw. Keratinozyten in einer bevorzugten Ausführungsform jeweils Thrombin und Calciumchlorid, das durch vorzugsweise simultane Zugabe von Fibrinogen beim Auftragen in die Wunde (z.B. mittels einer Zwillingsspritze) zu Fibrin reagiert und die Auspolymerisation der Matrix in der Wunde bewirkt. Dabei kann in einer bevorzugten Ausführungsform der vorliegenden Erfindung die Zelllösung von Anfang an als Mischung mit Fibrinogenlösung vorliegen und bei der Applikation in das Empfängerareal durch Thrombin/Calciumchloridzugabe aktiviert werden. In einer besonders bevorzugten Ausführungsform findet die Mischung der Fibrinogenlösung zu der Zellsuspension (die in diesem Fall bereits Thrombin und Calciumchlorid enthält) erst beim Aufbringen auf das Wundareal mittels Zwillingsspritze statt. Demzufolge umfasst die vorliegende Erfindung auch die Verwendung von Zwei-Komponentenzusammensetzungen bei denen die Fibrinmatrix und die Keratinozyten bzw. Fibroblastensuspensionen erst unmittelbar vor bzw. bei der Anwendung vereinigt werden.

Vorteilhafte Thrombinkonzentrationen liegen im Bereich von 50 bis 1000 i.E. Thrombin/ml, bevorzugt ist eine Konzentration von 200 bis 800 i.E./ml Lösung, besonders bevorzugt sind 500 i.E. Thrombin/ml. Vorteilhafte Calciumchloridkonzentrationen liegen im Bereich von 0.1 bis 50 mg/ml, bevorzugt ist 1 bis 10 mg/ml, besonders bevorzugt 5,88 mg/ml Calciumchlorid (alle Angaben stellen Endkonzentrationen dar). Eine ausführliche Darstellung von Fibrinklebern als biologische Zwei-Komponenten Systeme sowie deren Anwendung und Handhabung findet sich in EP 0 373 044, auf die hiermit in vollem Umfang Bezug genommen wird. Die verwendeten Kulturmedien sind auf den klinischen Einsatz am Menschen ausgerichtet.

Eine vorteilhafte Zelldichte der erfindungsgemäßen Zwei-Komponentenzusammensetzung beträgt sowohl für Bestandteil (a) als auch für Bestandteil (b) zwischen 0,5 bis 20 Millionen Zellen/ml Suspension, bevorzugt ist 1 bis 10 Millionen Zellen/ml, besonders bevorzugt 3 bis 6 Millionen Zellen/ml Suspension. Ein vorteilhaftes Zellmengenverhältnis der Bestandteile (a) zu (b) beträgt bei oberflächlichen Wunden 0,1:1 (a:b) bis 10:1 (a:b). Bevorzugt ist ein Zellmengenverhältnis von 0,5:1 bis 5:1 (a:b), besonders bevorzugt ist ein Verhältnis von 1:1. Bei tieferen Wunden ist ein Zellmengenverhältnis bevorzugt, daß einen Überschuß an Fibroblasten enthält, so daß ein besseres Auffüllen der profunden Wunde mit Granulationsmaterial ermöglicht wird. Der genaue jeweilige Ansatz der Suspensionskulturen hängt vom Einssatzzweck ab und liegt im Rahmen der Kenntnisse des Fachmanns auf dem Gebiet der Zellkultur (s. z.B. Hunyadi et al., J. Dermatol. Surg. Oncol., 1988, Vol. 14, Seiten 75-78; O'Connor und Mulliken, Lancet, 1981, Vol. 10, Seiten 75-78).

Die sowohl in Komponente (a) als auch (b) enthaltene Fibrinmatrix besteht in einer bevorzugten Ausführungsform der vorliegenden Erfindung aus einem handelsüblichen und arzneimittelrechtlich zugelassenen Fibrinkleber, wie z.B. "Tissucol Duo S Immuno" der Firma Baxter. Im Gegensatz zu den aus dem Stand der Technik bekannten (präformierten) Transplantaten auf Kollagenbasis wirkt die Fibrinmatrix bei Verwendung der Zwei-Komponmentenzusammensetzung fördernd auf die Migration, Proliferation und Differenzierung von Keratinozyten, Fibroblasten und Endothelzellen (Clark et al., J. Invest. Dermatol. (1982), Vol. 79, Seiten 264- 269; Tuan et al., Exp. Cell. Res. (1996), Vol. 223, Seiten 127-134; Ongenae et al., Dermatol. Surg. (2000), Vol. 26, Seiten 447- 451). Eine mögliche Erklärung für die o.g. Vorteile der erfindungsgemäßen Fibrinmatrix ist die Tatsache, daß die Anwesenheit von präformiertem Kollagen die Ausbildung weiterer dermaler Strukturen über einen *Feed-Back* Mechanismus hemmt (in der Art einer Produkthemmung), d.h. die Zellen erhalten bei Vorhandensein von Kollagen das Signal, die Proliferation herunterzuregulieren oder ganz einzustellen. Desweiteren wurde gezeigt, dass Fibroblasten in 3D-Kollagengelen apoptotisch werden (Fluck et al., J. Invest. Dermatol., 1998, Vol. 110, Seiten 153-157). Derartige Effekte sind jedoch für ein frisch eingebrachtes Transplantat von großem Nachteil, da eine Zellmigration und Proliferation unbedingt erforderlich ist, um ein Verwachsen und Integrieren des Transplantates in die Wunde zu ermöglichen und eine intakte dermo-epidermale Junktionszone auszubilden, die für ein stabiles physiologisches Transplantat erforderlich ist. Die erfindungsgemäße Verwendung der Zwei-Komponenten Zusammensetzung beseitigt dieses Problem und induziert physiologisches Granulationsgewebe, was zu einem physiologisch angepassten Matrixumbau durch die beteiligten Zelltypen selbst führt und somit eine verbesserte Textur des Unterhautgewebes sowie der dermo-epidermalen Junktionszone bewirkt. Da zur Wundheilung unter Verwendung der erfindungsgemäßen Zusammensetzung lediglich jeweils ein dünner Film der Komponenten (a) und (b) aufgetragen werden muß - in einer bevorzugten Ausführungsform findet flächendeckende Benetzung des Wundareals statt -, verringert sich gegenüber Transplantaten des Standes der Technik die Diffusionsstrecke für Nährstoffe, angiogenetische Faktoren und andere regulatorisch aktive Substanzen. Demzufolge stellt die erfindungsgemäße Zusammensetzung ein Zwei-Komponentensystem bereit, das die Transplantatqualität, dessen Lebensdauer und die physiologische Integration des transplantierten Gewebes gegenüber dem Stand der Technik deutlich verbessert.

Die vorliegende Erfindung betrifft des weiteren in einer bevorzugten Ausführungsform die Verwendung einer Zwei-Komponenten Zusammensetzung gemäß dem obigen Anspruch, bei der die Fibroblasten- und/oder die Keratinozytensuspension aus autologen Spenderzellen des zu behandelnden Patienten gewonnen wurden. Zu diesem Zweck wird dem Patienten zunächst ein ca. 1 bis 2 cm² großes Vollhautbiopsat entnommen, das anschließend in einer vorteilhaften Ausführungsform 2 bis 28 Tage in Kultur genommen wird, wobei 4 bis 21 Tage bevorzugt und 14 bis 18 Tage besonders bevorzugt ist. Mit 1 ml autologer thrombinhaltiger Keratinozyten-(b) bzw. Fibroblastenlösung (a), die in einer besonders bevorzugten Ausführungsform 500 i.E. humanes Thrombin enthalten, oder, in einer bevorzugten Ausführungsform, in Fibrinogenlösung vorliegen und beim Einbringen in das Empfängerareal mit Thrombin/Calciumchloridlösung gemischt werden.

Die Verwendung der Komponenten (a) und (b), die in einer besonders bevorzugten Ausführungsform der vorliegenden Erfindung 3 bis 6 x 10⁶ Zellen umfassen, lassen bei oberflächlichen Defekten die Transplantation von ca. 100 cm² Wundfläche zu. Sind tiefere Wunden zu transplantieren, so ist ein größeres Mengenverhältnis von Fibroblasten zu Keratinozyten vorteilhaft, wobei das genaue Verhältnis von der Tiefe der Wunde abhängt und im Einzelfall vom Transplanteur festgelegt werden muß.

Die vorliegende Erfindung betrifft in einer bevorzugten Ausführungsform ferner die Verwendung einer Zusammensetzung, bei der die verwendeten Fibroblasten- und Keratinozytensuspensionen im wesentlichen kollagenfrei sind. Dabei bedeutet "im wesentlichen kollagenfrei", dass kein Kollagen zugesetzt wird und bereits vorhandenes Kollagen durch Behandlung mit Kollagenase entfernt wird. In einer vorteilhaften Ausführungsform der vorliegenden Erfindung wird der Kollagengehalt der verwendeten Zellsuspensionen des Zwei-Komponentensystems (a und b) auf 40 bis 60% der ursprünglich in der Suspensionskultur vorhandenen Ausgangsmenge reduziert, in einer bevorzugten Ausführungsform wird der Kollagengehalt auf 20 bis 40% der Ausgangsmenge reduziert; besonders bevorzugt ist eine Reduktion des Kollagengehaltes auf unter 20% der Ausgangsmenge. Allgemein gilt, daß ein besonders niedriger Kollagengehalt (unter 20% der Ausgangsmenge) sich besonders vorteilhaft auf die physiologische Integration des Transplantates auswirkt.

Eine weitere bevorzugte Ausführungsform der vorliegenden Erfindung betrifft die Verwendung einer Zusammensetzung, bei der die verwendeten Fibroblasten- und/oder Keratinozytensuspensionen subkonfluent in Suspensionskultur vorliegen. Die verwendeten Suspensionskulturen enthalten - im Gegensatz zu Transplantaten des Standes der Technik, die auf konfluenten, präformierten Kulturen basieren - Zellen, die nicht kontaktinhibiert sind und die somit ein erhöhtes Migrations- und Proliferationspotential besitzen. Demzufolge stellt die Verwendung subkonfluent wachsender Fibroblasten bzw. Keratinozyten eine an die erfindungsgemäße Zwei-Komponenten Zusammensetzung angepasste Form der Kultur dar, die im Gegensatz zu präformierten Transplantaten eine biochemische und morphologische Anpassung des Transplantats an die Wunde ermöglicht, da die darin enthaltenen Zellen noch nicht vollständig ausdifferenziert sind. Demgemäss dient in einer bevorzugten Ausführungsform die Verwendung der Komponente a der erfindungsgemäßen Zusammensetzung zum flexiblen "aufgranulieren" profunder Wunden und Komponente b zum Abschluss bzw. zur Epithelbildung auf der Wundoberfläche.

In einer weiteren bevorzugten Ausführungsform betrifft die vorliegende Erfindung die Verwendung einer Zusammensetzung, bei der die Fibroblasten- und/oder die Keratinozytensuspension einen Fibrinkleber als Matrix enthält. In einer vorteilhaften Ausführungsform der vorliegenden Erfindung werden handelsübliche Fibrinkleber verwendet, die arzneimittelrechtlich zugelassen sind, wie z.B. das Produkt "Tissucol Duo S Immuno" der Firma Baxter.

In einer bevorzugten Ausführungsform der vorliegenden Erfindung werden kryokonservierte Zellen eingesetzt. Die Kryokonservierung dient dem Aufbewahren gewonnener Spenderzellen bzw. Zellen aus dem Spenderareal des Patienten, die nicht unmittelbar, sondern zu einem späteren Zeitpunkt transplantiert werden sollen. Die Kryokonservierung kann durchgeführt werden vor oder bei Erreichen der Subkonfluenz der jeweiligen Zellsuspension der Komponente (a) bzw. (b).

Weiterhin betrifft die vorliegende Erfindung die Verwendung einer Zusammensetzung, die ein von bovinem Serum freies Medium umfasst. Die Verwendung von Medien ohne bovines Serum stellt insoweit einen Vorteil dar, als das Infektionsrisiko, beispielsweise mit BSE bzw. der neuen Form der Creutzfeld-Jacob Krankheit, das nach derzeitigem Stand der Forschung nicht auszuschließen ist, minimiert wird. Verwendung finden stattdessen, wie bereits oben ausgeführt, bereits seit langer Zeit auf dem Markt etablierte Fibrinkleber bzw. -matrices. Des weiteren ist der bevorzugte Fibrinkleber frei von bovinem Kollagen.

Ferner können die bei der Verwendung der erfindungsgemäßen Zusammensetzung eingesetzten Fibroblasten- und Keratinozytensuspensionen gentechnisch modifizierte Zellen umfassen. Vorteilhaft sind in diesem Zusammenhang Zellen, in die mittels dem Fachmann bekannter Verfahren Gene bzw. Vektoren eingeschleust werden, die beispielsweise für chemotaktische und angiogenetische Faktoren, Hormone, Peptide oder Strukturproteine kodieren und somit den transplantierten Zellen wünschenswerte Eigenschaften verleihen können.

Die vorliegende Erfindung betrifft die Verwendung der erfindungsgemäßen Zusammensetzung in der Art, dass die Fibroblastensuspension (Komponente a) vor der Keratinozytensuspension (Komponente b) in die Wunde gebracht wird. In einer vorteilhaften Ausführungsform der vorliegenden Erfindung wird Komponente b wenige Sekunden bis Minuten nach der Komponente a) in die Wunde gebracht, in einer weiteren bevorzugten Ausführungsform beträgt dieser Zeitraum Minuten bis mehrere Stunden, besonders bevorzugt sind Zeiträume von mehrere Tagen. Für beide Komponenten a und b gilt, dass die Suspensionen, die zunächst Fibrinogen und die jeweiligen Zellen umfassen, unmittelbar bei der Applikation in die Wunde in einer bevorzugten Ausführungsform mit der Thrombin und Calciumchlorid enthaltenden Lösung gemischt werden, so dass die Polymerisation der Fibrinmatrix *in situ* stattfinden kann. In einer besonders bevorzugten Ausführungsform der vorliegenden Erfindung liegen die Zellen (Komponenten a und b) in Thrombin/Calciumchloridlösung vor und werden bei oder unmittelbar vor der Applikation in der Wunde erst zur endgültigen Zwei-Komponenten Zusammensetzung mit Fibrinogenlösung gemischt (mittels einer Zwillingsspritze).

Des weiteren betrifft die Erfindung die Verwendung einer erfindungsgemäßen Zusammensetzung, wobei die Fibroblastensuspension gemäß a) und/oder die Keratinozytensuspension gemäß b) vor der Verwendung mit einer Thrombinlösung gemischt werden. Für dieses Verfahren sind die für den oben genannten Verwendungsanspruch aufgeführten Zeitabstände bezüglich der Zugabe der Komponente (a) bzw. (b) bevorzugt.

Die Erfindung betrifft auch die Verwendung einer erfindungsgemäßen Zusammensetzung, wobei eine Thrombin enthaltende Fibroblastensuspension gemäß (a) und/oder eine Thrombin enthaltende Keratinozytensuspension gemäß (b) vor der Verwendung mit einer Fibrinogenlösung gemischt werden. Auch für dieses Verfahren sind die für den oben genannten Verwendungsanspruch aufgeführten Zeitabstände bezüglich der Zugabe der Komponente (a) bzw. (b) bevorzugt.

Ferner können im Rahmen dieser Verwendung die Fibroblastensuspension und/oder die Keratinozytensuspension vor der Herstellung der Komponenten (a) und (b) mit Kollagenase behandelt werden. Dies bedeutet, dass in den Zellsuspensionen bzw. -kulturen vorhandenes Kollagen durch Behandlung mit Kollagenase entfernt wird. In einer vorteilhaften Ausführungsform der vorliegenden Erfindung wird der Kollagengehalt der verwendeten Zellsuspensionen des Zwei-Komponentensystems (a und b) auf 40 bis 60% der ursprünglich in der Suspensionskultur vorhandenen Ausgangsmenge reduziert, in einer weiter bevorzugten Ausführungsform wird der Kollagengehalt auf 20 bis 40% der Ausgangsmenge reduziert; besonders bevorzugt ist eine Reduktion des Kollagengehaltes auf unter 20% der Ausgangsmenge. Allgemein gilt, wie bereits oben erwähnt, daß sich ein besonders niedriger Kollagengehalt (unter 20% der Ausgangsmenge) vorteilhaft auf die physiologische Integration des Transplantates in die zu therapierende Wunde bzw. Läsion auswirkt. Ferner ist im Rahmen der erfindungsgemäßen Verwendung eine Kultivierung der Fibroblastensuspension gemäß (a) und/oder der Keratinozytensuspension gemäß (b) unter Hypoxie möglich. Hartmann *et al*. fanden bereits 1997 bzw. 1999, dass unter Hypoxie kultivierte Zellen (hier: Endothelzellen bzw. Tumorzellen) eine Hochregulierung des Angiogeninspiegels aufwiesen (Hartmann et al., J. Invest. Dermatol. 1997, Vol. 109, Seite 438; Hartmann et al., Cancer Res., 1999, Vol. 59, Seiten 1578-1583). Im Falle der Tumorzellen wird so sichergestellt, dass die für die Versorgung und das Wachstum des Tumors unabdingbare verstärkte Angiogenese induziert wird. Diesen Effekt nutzt die vorliegende Erfindung, da das Schicksal des transplantierten Gewebes in besonderem Maße von einer schnellen Vaskularisierung des Wundareals abhängt. Somit führt die temporäre Hypoxie einer oder beider zu transplantierender Komponenten des Zwei-Komponenten Systems dazu, dass diese Zellen verstärkt angiogenetische Faktoren sezernieren und damit eine Vaskularisierung des Wundareals fördern. Die durch die Hypoxie initiierte Vaskularisierung stellt somit eine Anpassung des Gewebes bzw. der Zellen an eventuell auftretende Sauerstoffmangelzustände dar.

Ferner können der Fibroblastensuspension (Komponente a) und/oder der Keratinozytensüspension (Komponente b) angiogenetische Faktoren zugesetzt werden. Auch diese bevorzugte Ausführungsform der vorliegenden Erfindung basiert auf dem Gedanken, dass eine möglichst schnelle Vaskularisierung des transplantierten Areals wünschenswert und vorteilhaft ist. Als angiogenetische Faktoren kommen z.B. VEGF, Angiogenin und verwandte Peptidfaktoren in Frage. Die erforderliche Dosierung und Applikation dieser Faktoren kann vom Fachmann routinemäßig auf die jeweilige Anwendung abgestimmt werden.

Gemäß den voranstehend gemachten Ausführungen ist es im Rahmen der vorliegenden Erfindung gelungen, eine Zwei-Komponenten Zusammensetzung zur Verwendung in der Behandlung von Hautdefekten, Ulzera und Wunden bereitzustellen, die - im Gegensatz zu den präformierten Systemen des Standes der Technik - eine Transplantation von Fibroblasten und Keratinozyten erlaubt, die eine *in situ* Anpassung der subkonfluenten Zellen an das umgebende Wundgewebe erlaubt und sowohl die beschleunigte Bildung von Granulationsgewebe in der Wunde, als auch eine rasche Epithelbildung induziert. Somit können mit den vorliegenden Zusammensetzungen bzw. Verfahren zu deren Herstellung erstmals auch profunde Wunden transplantiert werden, ohne dass auf die körpereigene Granulation der Wunde gewartet werden muß. Die weiteren Nachteile der herkömmlichen Transplantate wie mechanische Instabilität und Infektionsgefahr werden ebenfalls beseitigt bzw. vermindert.

Die vorliegende Erfindung soll abschließend in Form eines Beispiels erläutert werden.

### Beispiel:

Eine dem Patienten entnommene Hautbiopsie wird nach Desinfektion in eine sterile Umgebung gebracht, in der die Bedingungen nach Reinheitsklasse A gemäß GMP-Richtlinien herrschen. Dies geschieht beispielsweise durch Übertragung der Probe in einen Arbeitsisolator mittels eines Transferisolators, wobei jeweils nahezu vollständige Keimfreiheit herrscht.

Zur Herstellung der Zellsuspensionen bzw. -kulturen für die Komponenten a und b werden folgende Schritte, vorzugsweise unter den in Klammern angegebenen Reinheitsbedingungen gemäß GMP-Standard, durchgeführt:
- Präparation der Hautbiopsie, mechanische Entfernung von Dermisresten (A)
- Spülung mit 70%-igem Ethanol und dann mit PBS-Puffer (A)
- Enzymatischer Verdau zur Ablösung der Epidermis von der Dermis (mindestens 2,4 U/ml Dispase, entweder 4 Stunden bei 37°C oder über Nacht bei 4°C) (D)
- Trennung der Epidermis von der Dermis durch "Abziehen" (A)
- Zerkleinerung der Epidermis in einer PBS-Pufferlösung mittels sterilen Instrumente, beispielsweise Scheren oder Pinzetten (A)
- Enzymatische Dissoziation einzelner Zellen durch Trypsinierung (0,5%-ige Trypsinlösung; 30 Minuten bei 37°C) (A oder D)
- Stoppen der Trypsinierung, beispielsweise durch Zugabe von Serum (A)
- Zentrifugation (zwischen 1500 und 4000 upm; bei Raumtemperatur; etwa 5 bis 10 Minuten) (D)
- Absaugen des Überstandes und Resuspension der Zellen in einem geeigneten Keratinozyten- bzw. Fibroblastennährmedium (z.B. in MEM- oder RPMI-Medium) (A)
- Aussäen der Zellen in Kulturflaschen (5000 - 50000 Zellen/cm²) (A)
- Kultivierung der Zellen bis zur Subkonfluenz (37°C, 5% CO₂, Mediumwechsel nach Bedarf, in der Regel jeden zweiten oder dritten Tag) (D)

Die Herstellung einer autologen Keratinozyten- (b) bzw. Fibroblastensuspension (a) der Zwei- Komponenten Zusammensetzung geschieht dann folgendermaßen:
- Enzymatische Dissoziation einzelner Zellen durch Trypsinierung (0,5%-ige Trypsinlösung; 30 Minuten bei 37°C) (A oder D)
- Stoppen der Trypsinierung, beispielsweise durch Zugabe von Serum (A)
- Zentrifugation (zwischen 1500 und 4000 upm; bei Raumtemperatur; etwa 5 bis 10 Minuten) (D)
- Resuspension der Zellen und Aufnahme der Zellen in Thrombin/Calciumchlorid- oder Fibrinogenlösung (4 Mio Zellen / 0,5 ml (A)).

Nicht in Thrombinlösung aufgenommene Zellen werden entweder sofort oder nach erneuter Weiterkultivierung bis zur Subkonfluenz kryokonserviert.

Vor der Auftragung der Zwei-Komponenten Zusammensetzung wird das Wundareal gereinigt und vorbereitet, z.B. durch Dermabrasion. Auf die entsprechenden Stellen wird zunächst die Suspension der autologen kultivierten Fibroblasten in Thrombin/Calciumchloridlösung aufgetragen (Komponente a), wobei die Verwendung einer Zwillingsspritze vorteilhaft ist, bei der eine Kammer die zu transplantierenden Fibroblasten in Thrombin/Calciumchloridlösung enthält und die andere Kammer Fibrinogenlösung, so dass eine Mischung der Zellen in Thrombin/Calciumchloridlösung mit Fibrinogen *in situ* stattfindet. Hierbei ist zu beachten, dass die Bildungsgeschwindigkeit des Fibrins aus Fibrinogen und damit die Gerinnung über die Thrombinkonzentration steuerbar ist und somit vom Fachmann die gewünschte Gerinnungszeit eingestellt werden kann.

Anschließend wird entweder direkt nach dem Auftragen der Komponente (a), bzw. einige Stunden oder Tage später (bezügl. der bevorzugten Zeiträumen s.o., Beschreibung) die Lösung (b), also Keratinozyten in Thrombin/Calciumchloridlösung aufgetragen, wobei sich ebenfalls in der zweiten Kammer einer Zwillingsspritze Fibrinogenlösung befindet und eine Mischung der Fibrinogenlösung mit den Zellen in Thrombin/Calciumchloridlösung unmittelbar vor oder bei der Applikation in das Empfängerareal stattfindet.

Nun befindet sich die vollständige Zwei-Komponenten Zusammensetzung im Empfängerareal, so dass eine physiologisch und morphologisch angepasste Regeneration des betroffenen Bereiches stattfinden kann.

## Patentansprüche

1. Zwei-Komponenten Zusammensetzung umfassend die folgenden Komponenten:
a) eine Fibroblastensuspension (Komponente a) enthaltend mindestens die getrennt vorliegenden Fibrinkleberbestandteile Thrombin und Calciumchlorid (Fibrinkleberbestandteil 1) und Fibrinogen (Fibrinkleberbestandteil 2) und
b) eine Keratinozytensuspension (Komponente b) enthaltend mindestens die getrennt vorliegenden Fibrinkleberbestandteile Thrombin und Calciumchlorid (Fibrinkleberbestandteil 1) und Fibrinogen (Fibrinkleberbestandteil 2)
zur Verwendung in der Behandlung von chronischen Ulzera unterschiedlicher Genese, von Verbrennungswunden, von posttraumatischen Wunddefekten, von postoperativen Wunddefekten nach Dermabrasion, Exzision oder Laser Ablation von Hautveränderungen oder in der Behandlung von nävoiden Hautveränderungen und von Narben nach Dermabrasion, **dadurch gekennzeichnet, dass** die Fibroblastensuspension gemäß a) vor der Keratinozytensuspension gemäß b) in die Wunde gebracht wird.

2. Zwei-Komponenten Zusammensetzung zur Verwendung nach Anspruch 1, bei der die Fibroblasten- oder die Keratinozytensuspension oder die Fibroblasten- und die Keratinozytensuspension aus autologen Spenderzellen des zu behandelnden Patienten gewonnen wurden.

3. Zwei-Komponenten Zusammensetzung zur Verwendung nach Anspruch 1 oder 2, bei der die verwendeten Fibroblasten- und Keratinozytensuspensionen im wesentlichen kollagenfrei sind.

4. Zwei-Komponenten Zusammensetzung zur Verwendung nach einem der Ansprüche 1 bis 3, bei der die Fibroblasten- oder die Keratinozytensuspension oder die Fibroblasten- und die Keratinozytensuspension subkonfluent in Suspensionskultur vorliegen.

5. Zwei-Komponenten Zusammensetzung zur Verwendung nach einem der Ansprüche 1 bis 4, die kryokonservierte Zellen umfasst.

6. Zwei-Komponenten Zusammensetzung zur Verwendung nach einem der Ansprüche 1 bis 5, wobei die Fibroblasten in Komponente a vor dem Mischen der beiden Fibrinkleberbestandteile entweder in dem Fibrinkleberbestandteil 1 oder in dem Fibrinkleberbestandteil 2 vorliegen.

7. Zwei-Komponenten Zusammensetzung zur Verwendung nach einem der Ansprüche 1 bis 6, wobei die Keratinozyten in Komponente b vor dem Mischen der beiden Fibrinkleberbestandteile entweder in dem Fibrinkleberbestandteil 1 oder in dem Fibrinkleberbestandteil 2 vorliegen.

8. Zwei-Komponenten Zusammensetzung zur Verwendung nach einem der Ansprüche 1 bis 7, wobei der Fibroblastensuspension gemäß a) oder der Keratinozytensuspension gemäß b) oder der Fibroblastensuspension gemäß a) und der Keratinozytensuspension gemäß b) angiogenetische Faktoren aufweisen.

## Claims

1. Two-component composition comprising the following components:
a) a fibroblast suspension (component a) containing at least the separately present fibrin adhesive components thrombin and calcium chloride (fibrin adhesive component 1) and fibrinogen (fibrin adhesive component 2) and
b) a keratinocyte suspension (component b) containing at least the separately present fibrin adhesive components thrombin and calcium chloride (fibrin adhesive component 1) and fibrinogen (fibrin adhesive component 2)
for use in the treatment of chronic ulcers of diverse origin, of burn wounds, of post-traumatic wound defects, of postoperative wound defects after dermabrasion, excision or laser ablation of skin changes or in the treatment of nevoid skin changes and of scars after dermabrasion, **characterized in that** the fibroblast suspension according to a) is introduced into the wound before the keratinocyte suspension according to b).

2. Two-component composition for use according to Claim 1, **characterized in that** the fibroblast or the keratinocyte suspension or the fibroblast and the keratinocyte suspension are obtained from autologous donor cells of the patient to be treated.

3. Two-component composition for use according to Claim 1 or 2, **characterized in that** the fibroblast and keratinocyte suspensions used are essentially collagen-free.

4. Two-component composition for use according to one of Claims 1 to 3, **characterized in that** the fibroblast or the keratinocyte suspension or the fibroblast and the keratinocyte suspension are present subconfluent in suspension culture.

5. Two-component composition for use according to one of Claims 1 to 4, **characterized in that** it contains cryopreserved cells.

6. Two-component composition for use according to one of Claims 1 to 5, **characterized in that** the fibroblasts in component a before the mixing of the two fibrin adhesive components are present either in the fibrin adhesive component 1 or in the fibrin adhesive component 2.

7. Two-component composition for use according to one of Claims 1 to 6, **characterized in that** the keratinocytes in component b before the mixing of the two fibrin adhesive components are present either in the fibrin adhesive component 1 or in the fibrin adhesive component 2.

8. Two-component composition for use according to one of Claims 1 to 7, **characterized in that** the fibroblast suspension according to a) or the keratinocyte suspension according to b) or the fibroblast suspension according to a) and the keratinocyte suspension according to b) contain angiogenic factors.

## Revendications

1. Composition à deux composants, comprenant les composants suivants :
a) une suspension de fibroblastes (composant a) contenant au moins les constituants adhésifs de la fibrine se trouvant sous forme séparée, la thrombine et le chlorure de calcium (constituant adhésif de fibrine 1) et du fibrinogène (constituant adhésif de fibrine 2) et
b) une suspension de kératinocytes (composant b) contenant au moins les constituants adhésifs de la fibrine se trouvant sous forme séparée, la thrombine et le chlorure de calcium (constituant adhésif de fibrine 1) et du fibrinogène (constituant adhésif de fibrine 2)
pour une utilisation dans le traitement d'ulcères chroniques de différentes genèses, de brûlures, de défauts de plaies post-traumatiques, de défauts de plaies post-opératives après une dermabrasion, une excision ou une ablation par laser de modifications de la peau ou dans le traitement de modifications naevoïdes de la peau et de cicatrices après une dermabrasion, **caractérisée en ce que** la suspension de fibroplastes selon a) est introduite dans la plaie avant la suspension de kératinocytes selon b).

2. Composition à deux composants pour une utilisation selon la revendication 1, dans laquelle la suspension de fibroblastes ou la suspension de kératinocytes ou la suspension de fibroblastes et la suspension de kératinocytes est obtenue à partir de cellules donneuses autologues du patient à traiter.

3. Composition à deux composants pour une utilisation selon la revendication 1 ou 2, dans laquelle les suspensions utilisées de fibroblastes et de kératinocytes sont essentiellement exemptes de collagène.

4. Composition à deux composants pour une utilisation selon l'une quelconque des revendications 1 à 3, dans laquelle la suspension de fibroblastes ou la suspension de kératinocytes ou la suspension de fibroblastes et la suspension de kératinocytes se trouvent sous forme sous-confluente dans la culture en suspension.

5. Composition à deux composants pour une utilisation selon l'une quelconque des revendications 1 à 4, qui comprend des cellules cryoconservées.

6. Composition à deux composants pour une utilisation selon l'une quelconque des revendications 1 à 5, où les fibroblastes dans le composant a se trouvent, avant le mélange des deux constituants adhésifs de fibrine, soit dans le constituant adhésif de fibrine 1, soit dans le constituant adhésif de fibrine 2.

7. Composition à deux composants pour une utilisation selon l'une quelconque des revendications 1 à 6, où les kératinocytes dans le composant b se trouvent, avant le mélange des deux constituants adhésifs de fibrine, soit dans le constituant adhésif de fibrine 1, soit dans le constituant adhésif de fibrine 2.

8. Composition à deux composants pour une utilisation selon l'une quelconque des revendications 1 à 7, où la suspension de fibroblastes selon a) ou la suspension de kératinocytes selon b) ou la suspension de fibroblastes selon a) et la suspension de kératinocytes selon b) présentent des facteurs angiogénétiques.
